(19) Europäisches Patentamt / European Patent Office / Office européen des brevets

(11) **EP 4 345 451 B1**

(12) **EUROPEAN PATENT SPECIFICATION**

(45) Date of publication and mention
of the grant of the patent:
**08.04.2026  Bulletin 2026/15**

(51) International Patent Classification (IPC):
*G01N 30/72* *(2006.01)*    *G01N 30/86* *(2006.01)*
*G01N 30/88* *(2006.01)*

(21) Application number: **23196728.2**

(52) Cooperative Patent Classification (CPC):
**G01N 30/88; G01N 30/7206; G01N 30/8675;**
G01N 2030/884

(22) Date of filing: **12.09.2023**

(54) **ANALYZER, ANALYSIS METHOD, AND ANALYSIS PROGRAM FOR RUBBER COMPOSITION**

ANALYSEGERÄT, ANALYSEVERFAHREN UND ANALYSEPROGRAMM FÜR EINE
KAUTSCHUKZUSAMMENSETZUNG

ANALYSEUR, PROCÉDÉ D'ANALYSE ET PROGRAMME D'ANALYSE POUR UNE COMPOSITION
DE CAOUTCHOUC

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: **28.09.2022  JP 2022155012**
**05.12.2022  JP 2022194087**

(43) Date of publication of application:
**03.04.2024  Bulletin 2024/14**

(73) Proprietors:
• **Sumitomo Rubber Industries, Ltd.**
**Kobe-shi, Hyogo 651-0072 (JP)**
• **NEC Corporation**
**Tokyo 108-8001 (JP)**

(72) Inventors:
• **YOSHITANI, Mio**
**Kobe-shi, Hyogo, 651-0072 (JP)**
• **UNNO, Yuma**
**Kobe-shi, Hyogo, 651-0072 (JP)**
• **YAMADA, Hiroaki**
**Kobe-shi, Hyogo, 651-0072 (JP)**
• **ISHIZAWA, Yoshio**
**Tokyo, 108-8001 (JP)**
• **KONDO, Takashi**
**Tokyo, 108-8001 (JP)**
• **KUSAKA, Junichiro**
**Tokyo, 108-8001 (JP)**

(74) Representative: **Manitz Finsterwald**
**Patent- und Rechtsanwaltspartnerschaft mbB**
**Martin-Greif-Straße 1**
**80336 München (DE)**

(56) References cited:
CN-A- 113 176 354    CN-A- 114 043 651
CN-A- 114 705 793    JP-A- 2013 160 599
JP-A- 2017 181 284    JP-B2- 6 592 985

• **CHEN XIN ET AL: "Traceability of VOCs in tire
inner liner by chromatography-mass
spectrometry", ENVIRONMENTAL SCIENCE AND
POLLUTION RESEARCH, SPRINGER BERLIN
HEIDELBERG, BERLIN/HEIDELBERG, vol. 29,
no. 7, 9 September 2021 (2021-09-09), pages 9685
- 9692, XP037672698, ISSN: 0944-1344, [retrieved
on 20210909], DOI: 10.1007/S11356-021-16284-1**

**Description**

FIELD OF INVENTION

The present invention relates to an analysis method, an analyzer, and an analysis program for a rubber composition, and a computer-readable medium having stored thereon the analysis program.

BACKGROUND

JP 2013-160599 A discloses an analysis method for a rubber composition based on a pyrolysis gas chromatography/mass spectrometry (GC-MS) method. According to JP 2013-160599 A, a thermal decomposition unit decomposes a rubber material. A gas chromatography unit separates a decomposition product. A mass spectrum unit ionizes the decomposition product with an electron beam. A detector detects the ionized decomposition product. A total ion chromatogram and mass spectrums are thus acquired. According to JP 2013-160599 A, peaks in the acquired total ion chromatogram and the acquired mass spectrums are analyzed. Based on the acquired peaks, qualitative and quantitative analyses are performed. The rubber composition is thus analyzed for its composition.

Furthermore, JP 6592985 B2 discloses a method for quantitatively evaluating natural rubber for its odor. With this method, a content of a certain component in natural rubber is quantified using a gas chromatograph, for example. Based on the acquired content of the certain component, an odor index for the certain component is thus derived. By summing the derived odor indexes, it is thus possible to objectively evaluate the natural rubber in question for its odor. CN 114043651 A and X. Chen et al., Environ. Sci. Pollut. Res. (2022) 29:9685-9692 disclose the determination of the VOC amount in a rubber sample by means of GC-MS. CN 113176354 A describes a GC-MS method wherein volatile odor substances from wood samples are detected.

SUMMARY of INVENTION

The analysis proposed in JP 2013-160599 A is performed using existing analysis software and through visual observation based on charts of a total ion chromatogram and mass spectrums that a gas chromatograph-mass spectrometer outputs. However, such a method is subjected to peaks derived from known components and relatively large peaks. Thus, information about peaks derived from unknown components and relatively small peaks have not been fully utilized. Therefore, it has been difficult to accurately estimate a physical property of a rubber composition based on such charts as described above. With the method proposed in JP 6592985 B2, on the other hand, even when there is an odor component that contributed to odor in a target to be analyzed in addition to a certain component that is determined beforehand, it is not possible to take into account those peaks derived from the odor component. Therefore, there has been a room for improvement in accuracy of estimating an odor degree.

An object of the present disclosure is to provide an analysis method, an analyzer, and an analysis program for accurately estimating, based on data acquired by allowing a rubber composition to undergo component separation and mass separation, at least either of a physical property and an odor degree of the rubber composition.

The above-mentioned problems are solved by an analysis method according to claim 1, which is an analysis method executed by one or more processors, including those described below.

(1) Acquiring data acquired by allowing a rubber composition to undergo component separation and mass separation, the data being plotted in a space defined by a time axis, a signal intensity axis, and a mass component axis.
(2) Identifying peaks in signal intensity for mass chromatograms corresponding to positions on the mass component axis;

performing grouping within a range designated on the time axis for the peaks identified for the mass chromatograms;
extracting a desired pair of the peaks having undergone the grouping, determines, when an index indicating a deviation between the peaks constituting the pair is equal to or lower than a predetermined threshold or below the threshold, the peaks constituting the pair as peaks derived from components that are identical to each other, and determines, when the index is above the threshold or equal to or higher than the threshold, the peaks constituting the pair as peaks derived from components that differ from each other.

(3) Estimating at least either of a physical property and an odor degree of the rubber composition based on the peaks identified for the mass chromatograms. An analyzer for executing the analysis method for a rubber composition of the present invention, according to a first aspect, includes an acquisition unit, a peak identification unit, and an estimation unit. The acquisition unit acquires data acquired by allowing a rubber composition to undergo component separation and mass separation, the data being plotted in a space defined by a time axis, a signal intensity axis, and a mass component axis. The peak identification unit identifies peaks in signal intensity for mass chromatograms corresponding to positions on the mass component axis. The estimation unit estimates at least either of a physical property and an odor degree of the rubber composition based on the peaks identified for the mass chromatograms.

An analyzer for a rubber composition, according to a second aspect is the analyzer for a rubber composition according to the first aspect, in which the peak identification unit performs grouping within a range designated on the time axis for the peaks identified for the mass chromatograms.

An analyzer for a rubber composition according to a third aspect is the analyzer for a rubber composition according to the second aspect, in which the acquisition unit further acquires a chromatogram acquired by summing the signal intensities of the mass chromatograms that are present at an identical position on the time axis. The peak identification unit designates a range of the grouping to be performed based on the peaks identified in the chromatograms.

An analyzer for a rubber composition, according to a fourth aspect is the analyzer for a rubber composition according to the second aspect or the third aspect, in which the peak identification unit extracts a desired pair of the peaks having undergone the grouping, determines, when an index indicating a deviation between the peaks constituting the pair is equal to or lower than a predetermined threshold or below the threshold, the peaks constituting the pair as peaks derived from components that are identical to each other, and determines, when the index is above the threshold or equal to or higher than the threshold, the peaks constituting the pair as peaks derived from components that differ from each other.

An analyzer for a rubber composition, according to a fifth aspect is the analyzer for a rubber composition according to the fourth aspect, in which the estimation unit estimates at least either of a certain physical property and an odor degree of the rubber composition based on areas determined by the peaks identified by the peak identification unit to be derived from the components that are identical to each other.

An analyzer for a rubber composition, according to a sixth aspect is the analyzer for a rubber composition according to the fifth aspect, in which the estimation unit estimates at least either of a certain physical property and an odor degree of the rubber composition without identifying the components that have derived the peaks.

An analyzer for a rubber composition according to a seventh aspect is the analyzer for a rubber composition according to any one of the first aspect to the sixth aspect, in which the physical property includes a glass transition temperature.

An analyzer for a rubber composition according to an eighth aspect is the analyzer for a rubber composition according to any one of the first aspect to the seventh aspect, in which the data is acquired by a gas chromatograph-mass spectrometer.

An analyzer for a rubber composition according to a ninth aspect is the analyzer for a rubber composition according to the eighth aspect, in which the data is acquired by a head space gas chromatograph-mass spectrometer.

An analysis program for a rubber composition according to an eleventh aspect causes one or more processors to execute the analysis method according to the invention. Furthermore, the present invention provides a computer-readable medium having stored thereon the analysis program for a rubber composition.

According to the aspects described above, an analysis method, an analyzer, and an analysis program for accurately estimating, based on data acquired by allowing a rubber composition to undergo component separation and mass separation, at least either of a physical property and an odor degree of the rubber composition are provided.

BRIEF DESCRIPTION OF DRAWINGS

FIG. 1 is a block diagram illustrating an electrical configuration of an analyzer.
FIG. 2 is a diagram illustrating data of a rubber composition.
FIG. 3 is a flowchart illustrating a flow of analysis processing according to an embodiment.
FIG. 4 is a diagram illustrating designation processing for a grouping range.
FIG. 5 is a diagram illustrating identification processing for a peak.
FIG. 6 is a diagram illustrating summing processing for peaks.
FIG. 7 is a diagram illustrating an estimation index according to the embodiment.
FIG. 8 is an example of a peak list.

DETAILED DESCRIPTION OF THE PREFERRED EMBODIMENTS

An analyzer, an analysis method, and an analysis program for a rubber composition, according to an embodiment of the present disclosure, will now be described herein.

<1. Analyzer>

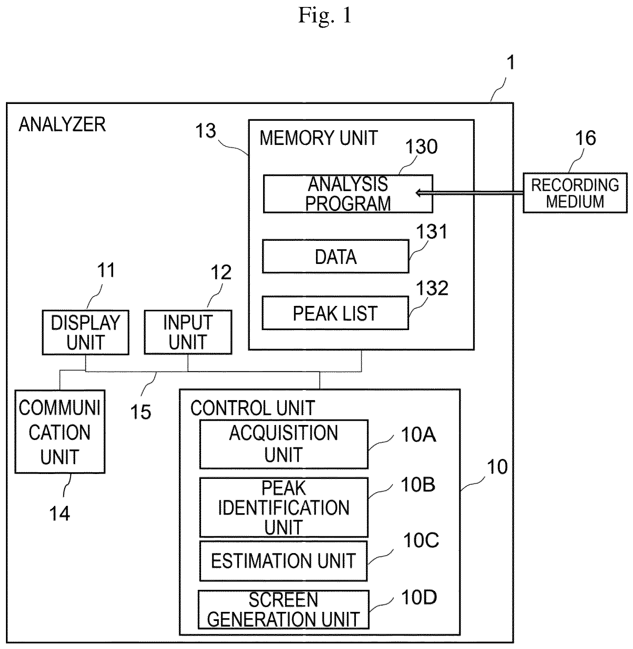

FIG. 1 is a block diagram illustrating an electrical configuration of an analyzer 1. The analyzer 1 is a device that analyzes data acquired by allowing a rubber composition to undergo component separation and mass separation in accordance with an algorithm defined by an analysis program 130, and estimates at least either of a physical property and an odor degree of the rubber composition.

Data

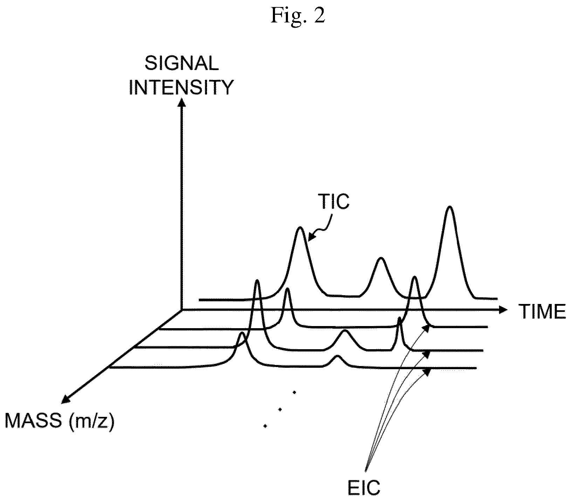

The data described above is, in the present embodiment, data acquired by allowing a sample rubber composition that is a target to be analyzed to be subjected to a gas chromatograph (GC) to undergo component separation, by allowing a chemical compound having undergone the component separation to be subjected to a mass spectrometer (MS) to undergo mass separation, and by detecting the chemical compound having undergone the component separation and mass separation. More specifically, the data described above is, as illustrated in FIG. 2, three-dimensional data plotted in a space defined by a time axis, a signal intensity axis, and a mass component axis. The time axis indicates a retention time that has passed after a sample is introduced into the gas chromatograph. The signal intensity axis indicates a signal intensity outputted by a detector of the mass spectrometer. A peak in the signal intensity means that a component is detected. Furthermore, in the mass spectrometer, a chemical compound derived from a sample is normally ionized and detected. Therefore, the mass component axis in the present embodiment indicates a mass/charge ratio (m/z) of ions. Note that it is possible to appropriately set a range of the values of m/z, across which the mass spectrometer scans, and an interval in an axial direction.

The three-dimensional data described above is acquired by a known gas chromatograph-mass spectrometer. Furthermore, the acquired three-dimensional data is outputted, by an analysis program that the gas chromatograph-mass spectrometer stores, in a chart format illustrating a signal intensity, which corresponds to a position of the value of m/z on the mass component axis, with respect to a point in time. The chart is also referred to as a "mass chromatogram" or an "extracted ion chromatogram (EIC)". The chart is generated per one interval of the value of m/z in the present embodiment.

A total ion chromatogram (TIC) is one acquired by summing the mass chromatograms described above within a whole range of the values of m/z being set. That is, a TIC is a chromatogram acquired by summing the signal intensities in the mass chromatograms that are present at an identical position on the time axis. A TIC is normally generated and outputted by a known analysis program. Note that peaks in signal intensity, which appear in a TIC, include both of peaks derived from a known components and peaks derived from unknown components.

There is no particular limitation in gas chromatograph, as long as gas that is a target to be analyzed is fed into a column together with carrier gas, and a component of the gas, which is generated during a period of time where the gas passes through the column, is separated. It is possible to use a known gas chromatograph. There is also no particular limitation in pretreatment before the gas that is the target to be analyzed is fed into the column. For example, it is possible to adopt any of those including: a method of generating a thermal decomposition product acquired from a sample using a thermal decomposition device as a chemical compound having undergone component separation; a method of heating a sample in a head space sampler for a constant period of time to allow a gas phase and the sample to be in an equilibrium state; and a method of heating a sample in a sample tube to generate a component, of collecting the generated component in a cold trap, and of reheating the collected component for desorption. Therefore, a gas chromatograph used in here may be any of a pyrolysis gas chromatograph, a head space-gas chromatograph (HS-GC), and a thermal desorption-gas chromatograph (TDU-GC), for example. Note that, when estimating an odor degree of a rubber composition, it is preferable to use an HS-GC and a TDU-GC, which offer less thermal decomposition of chemical compounds. It is more preferable to use an HS-GC. Furthermore, there is also no particular limitation in mass spectrometer, as long as molecular ions and fragment ions generated through ionization of gas that has passed through a gas chromatograph are separated and detected based on a difference in the value of m/z. It is thus possible to use a known mass spectrometer.

Analyzer

The analyzer 1 is a general-purpose computer in terms of hardware, and is realized as a desktop personal computer, a laptop personal computer, a tablet, or a smartphone, for example. The analyzer 1 is manufactured by, for example, installing the analysis program 130 into a general-purpose computer from a recording medium 16 that is readable by a computer, such as a compact disc-read only memory (CD-ROM) or a universal serial bus (USB) memory, or via a network.

The analyzer 1 includes a control unit 10, a display unit 11, an input unit 12, a memory unit 13, and a communication unit 14. The units 10 to 14 are coupled to each other via a bus line 15, and are thus communicable with each other. Furthermore, at least some of the display unit 11, the input unit 12, and the memory unit 13 may be integrally incorporated into a main body of the analyzer 1 (a housing accommodating the control unit 10 and other components), or may be externally-attached units.

It is possible to configure the display unit 11 using, for example, a liquid crystal display, an organic electroluminescence (EL) display, a plasma display, or a liquid crystal element, to display various types of information to a user. It is possible to configure the input unit 12 using, for example, a mouse. a keyboard, and a touch panel, to receive maneuvers of the user to the analyzer 1. The communication unit 14 functions as a communication interface that establishes communication connections in various types of formats.

It is possible to configure the control unit 10 using processors such as a central processing unit (CPU) and a graphics processing unit (GPU), a read-only memory (ROM), and a random access memory (RAM). The control unit 10 reads and executes the analysis program 130 in the memory unit 13 to operate in a virtual manner as an acquisition unit 10A, a peak identification unit 10B, an estimation unit 10C, and a screen generation unit 10D. The acquisition unit 10A reads such three-dimensional data as described above from an external device through data communications via the communication unit 14, or via the recording medium 16, and stores the read three-dimensional data as "data 131" in the memory unit 13 or the RAM. The peak identification unit 10B uses a method described later to identify peaks included in the data 131, lists the identified peaks into a piece of data (hereinafter also referred to as a "peak list 132"), and stores the data in the memory unit 13 or the RAM. The estimation unit 10C estimates at least either of a physical property and an odor degree of the rubber composition in question based on the peak list 132. The screen generation unit 10D generates a screen displaying a result of the estimation by the estimation unit 10C. The functions of the units 10A to 10D are realized by one or more processors.

It is possible to configure the memory unit 13 using a nonvolatile memory device such as a hard disk or a flash memory. In addition to the analysis program 130, the memory unit 13 stores the data 131 acquired by the acquisition unit 10A and the peak list 132 created by the peak identification unit 10B.

<2. Operation of analyzer>

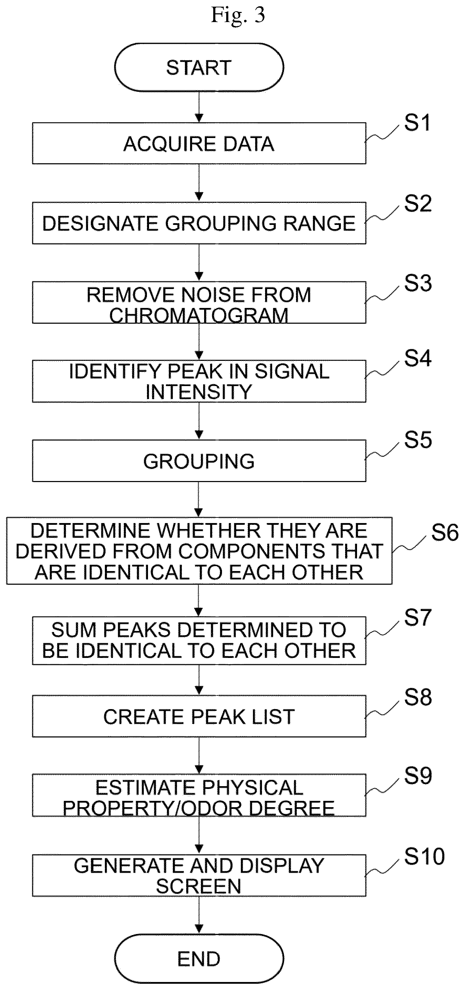

FIG. 3 is a flowchart illustrating a flow of an analysis method for the data 131, which is executed by the analyzer 1. The processing in FIG. 3 starts, for example, when an instruction for starting analysis processing is inputted by the user via the input unit 12 to the analyzer 1, and is received by the control unit 10.

The acquisition unit 10A first acquires data acquired by allowing a rubber composition that is a target to be analyzed to undergo component separation and mass separation, and stores the acquired data as the data 131 in the memory unit 13 or the RAM (step S1). As described above, the data is three-dimensional data to be outputted from the gas chromatograph-mass spectrometer, and includes data of a total ion chromatogram and mass chromatograms of mass components. There is no particular limitation in data acquisition method. For example, data may be read from a gas chromatograph-mass spectrometer through wired or wireless data communications. Data may be acquired from a recording medium that is storing the data.

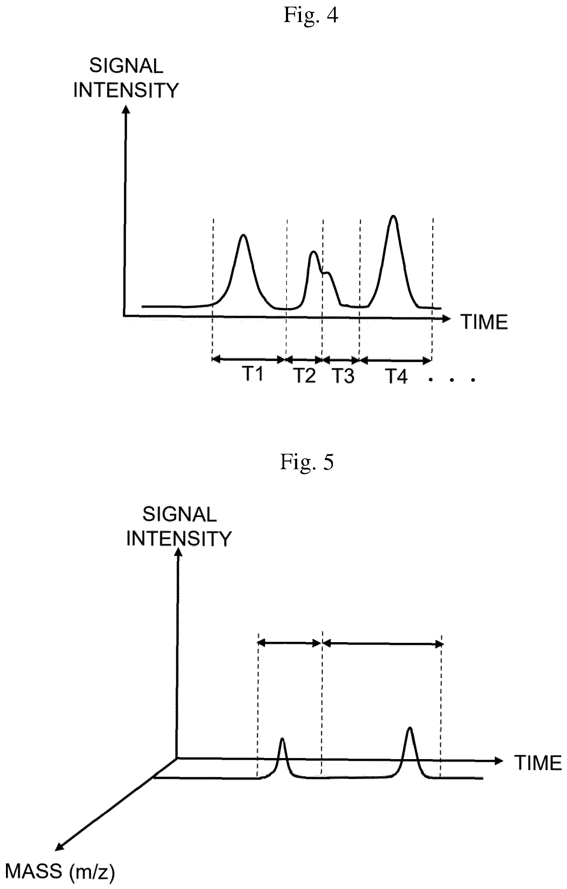

The peak identification unit 10B then reads the TIC from the data 131 and designates grouping ranges T1, T2, T3, T4, etc. based on peaks identified on the chart (step S2). A grouping range is a range on the time axis. The grouping range is designated when performing grouping of peaks in mass chromatograms at step S5 described later. The peak identification unit 10B identifies, in the TIC, as illustrated in FIG. 4, a point in time when a first peak rises and a point in time when there is the minimum signal intensity between peaks adjacent to each other. Intervals of the identified points in time serve as the grouping ranges T1, T2, etc. It is possible to use a known analysis method to identify a point in time when the first peak rises and a point in time when there is the minimum signal intensity between peaks adjacent to each other. Note that it is preferable that each of the grouping ranges T1, T2, etc. designated in here includes the top of one peak. However, a grouping range may include the tops of two or more peaks. That is, the grouping ranges T1, T2, etc. may be designated based on two or more peaks that have not yet been separated on a TIC.

The peak identification unit 10B then reads the mass chromatograms for the mass components from the data 131, and allows all the mass chromatograms to undergo noise removal processing (step S3). The noise removal processing is processing for defining, for each of the mass chromatograms, one having a largest absolute value, among negative signal intensities, as a noise width in the mass chromatogram, and for disregarding a peak where the signal intensity at its top is below the noise width or equal to or narrower than the noise width as a peak that is subjected to next and subsequent processing, that is, for regarding it as noise to be removed. In other words, the noise removal processing is processing for selecting a peak where the signal intensity at its top is equal to or wider than the noise width or above the noise width as a peak that is subjected to next and subsequent processing. Note that there is no limitation in noise width definition method. It is possible to appropriately change a width definition method. For example, for each of the mass chromatograms, a time range with no peak may be designated to create a signal intensity histogram. A value acquired by multiplying a width of Gaussian distribution, which is assumed for the created signal intensity histogram, by a predetermined coefficient may be defined as a noise width in the mass chromatogram.

The peak identification unit 10B then identifies a peak in signal intensity for each of the mass chromatograms having undergone the noise removal processing at step S3 (step S4). More specifically, for each of mass chromatograms corresponding to positions on the mass component axis, similar to step S2, a point in time when the first peak rises and a point in time when there is the minimum signal intensity between peaks adjacent to each other, are identified. A time range for each of the peaks is then identified based on the identified point in time when the first peak rises and the identified point in time when there is the minimum signal intensity between peaks adjacent to each other (see FIG. 5). It is possible to identify a peak using a known analysis method. Furthermore, for identification of peaks, it is not restricted to the grouping ranges T1, T2, etc. designated at step S2. A peak is identified for each of mass chromatograms.

Next, the peak identification unit 10B performs grouping for the peaks identified in each of the mass chromatograms in terms of each of the grouping ranges T1, T2, etc., to which the peaks belong (step S5). In addition to this grouping, the peak identification unit 10B may perform screening based on the signal intensities at the tops, for the peaks in all the mass chromatograms, which belong to each of the grouping ranges, to reduce, in number, peaks that are subjected to next and subsequent steps. It is possible that the screening is, for example, processing for adopting a predetermined number of peaks in an order beginning from the largest signal intensity at the top among peaks belonging to an identical grouping range, while removing other peaks. Furthermore, it is possible that processing, for example, is for adopting peaks each having the signal intensity at its top, which is equal to or above a predetermined ratio, for maximum values of the signal intensities at the tops, among peaks belonging to an identical grouping range, while removing peaks each having the signal intensity at its top, which is below the predetermined ratio.

The peak identification unit 10B then determines, for respective peaks in the mass chromatograms, which are allocated in an identical grouping range at step S5, whether the peaks correspond to peaks derived from components that are identical to each other (step S6). In the present embodiment, this determination is performed based on an index indicating a deviation in a pair of desired peaks belonging to an identical grouping range. For example, when there are peaks (A, B, C, and D) belonging to an identical grouping range, the peak identification unit 10B extracts pairs of all of them (six pairs of A and B, A and C, A and D, B and C, B and D, and C and D). For some or all of the pairs, indexes $\Delta V$ each indicating a deviation between two ones of the peaks are then calculated. The indexes $\Delta V$ are each compared with a threshold V1 determined beforehand. The peak identification unit 10B determines, when the index $\Delta V$ is equal to or lower than the threshold V1 or below the threshold V1, the peaks constituting the pair as peaks derived from components that are identical to each other, and determines, when the index $\Delta V$ is above the threshold V1 or equal to or higher than the threshold V1, the peaks constituting the pair as peaks derived from components that differ from each other. By performing this determination for all pairs belonging to a certain grouping range, it is possible to more finely separate peaks belonging to the grouping range.

It is assumed in here that the indexes $\Delta V$ for all the pairs in the example described above are calculated, and, as a result of comparing each of the indexes with the threshold V1, the pairs of A and B, A and C, and B and C are determined to be derived from components that are identical to each other, while the pairs of A and D, B and D, and C and D are determined to be derived from components that differ from each other. By summarizing the determinations, the peaks (A, B, and C) are determined to be derived from components that are identical to each other, while the peak (D) is determined to be derived from a component that differs from the components that have derived the other peaks. Note that when there are three or more peaks belonging to an identical grouping range, determinations based on the indexes $\Delta V$ calculated for the pairs may indicate a result where there is no consistency among the pairs. An example of such a case is when the indexes $\Delta V$ calculated for the pair of A and B and the pair of A and C are both equal to or lower than the threshold V1 (or below the threshold V1), while the index $\Delta V$ calculated for the pair of B and C is above the threshold V1 (or equal to or higher than the threshold V1). In such a case, determinations for the pair of A and B and the pair of A and C may be prioritized. It may be finally determined that the peaks (A, B, and C) are derived from components that are identical to each other. That is, a determination of whether peaks constituting a pair correspond to peaks derived from components that are identical to each other may be comprehensively performed by taking into account determinations for other pairs belonging to an identical grouping range. Alternatively, for three or more peaks belonging to an identical grouping range, the indexes $\Delta V$ are first calculated for different pairs that include an identical peak (in the example described above, the pair of A and B and the pair of A and C), respectively. When the peaks constituting the pairs are determined to be derived from components that are identical to each other based on the indexes $\Delta V$, a calculation of the index $\Delta V$ for the remaining pair (in the example described above, the pair of B and C) may be omitted.

The index $\Delta V$ is, for example, a mean absolute error (MAE) in data included in each peak at an identical point in time. When pieces of time-series data included in peaks constituting a pair are $y_{1i}$ ($i = 1,..., N$) and $y_{2i}$ ($i = 1,..., N$), respectively, a MAE is expressed by a formula described below. N is the number of pieces of data defined by a grouping range designated at step S2.

[Mathematical formula 1]

$$MAE = \frac{1}{N} \sum_{i=1}^{N} |y1i - y2i|$$

It is indicated that the larger the MAE serving as the index ΔV, the larger the deviation in shape between peaks constituting a pair. It is also indicated that the smaller the MAE, the smaller the deviation in shape between peaks constituting a pair. Therefore, it is indicated that the shapes are similar to each other. In addition to or instead of the index ΔV, it is possible that the peak identification unit 10B calculates an index ΔRT as an index indicating a deviation in position on the time axis between peaks. The index ΔRT indicates an absolute value of a difference in point in time at the top between peaks. It is thus indicated that the larger the absolute value of the index ΔRT, the larger the deviation in position between peaks constituting a pair, indicating that the positions are farther from each other. It is also indicated that the smaller the absolute value of the index ΔRT, the smaller the deviation in position between peaks constituting a pair, indicating that the positions are closer to each other. Similar to the index ΔV, it is possible to determine that, when the index ΔRT is equal to or lower than a threshold RT1 determined beforehand or below the threshold RT1, the peaks constituting a pair correspond to peaks derived from components that are identical to each other. It is also possible to determine that, when the index ΔRT is above the threshold RT1 or equal to or higher than the threshold RT1, the peaks constituting a pair correspond to peaks derived from components that differ from each other. Note that, if a plurality of indexes are used in a combined manner, it is conceivable that there may be no consistency in results of determinations based on each of the indexes. Therefore, a priority order (weight) may be set beforehand for a plurality of indexes, for example. Whether peaks constituting a pair are derived from components that are identical to each other may be determined in accordance with the weight that is set per index.

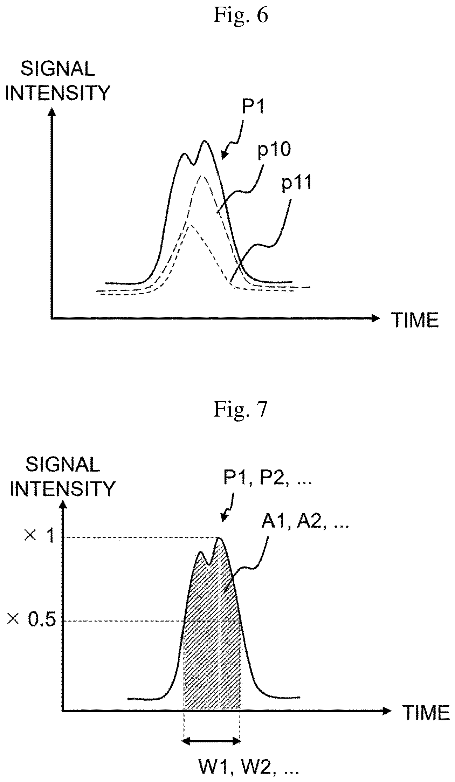

The peak identification unit 10B performs summing for each of groups of peaks determined to be derived from components that are identical to each other at step S6 (i.e., which appear at an identical position on the time axis), and generates peaks P1, P2, etc. that are conceivable to be derived from components that are identical to each other (step S7). More specifically, the peak identification unit 10B identifies all peaks that are conceivable to be present at an identical position on the time axis, but that are present at positions that differ from each other on the mass component axis, and combines the peaks to generate one peak at an identical point in time. When two peaks p10 and p11 are identified, which are determined to be derived from components that are identical to each other, as illustrated in FIG. 6, for example, the peak identification unit 10B sums the peaks to generate the peak P1. The peak P2 and subsequent peaks are also generated in the same manner. The peak identification unit 10B associates the generated peaks P1, P2, etc., the positions of the peaks on the time axis, and the values of m/z of the peaks from which the peaks P1, P2, etc. are derived with each other, sorts the peaks in order on the time axis, and creates a peak list storage area for storing the peak list 132 in the memory unit 13 or the RAM.

The estimation unit 10C then calculates an estimation index for estimating a physical property or an odor degree of the rubber composition for each of the peaks P1, P2, etc., and creates the peak list 132 (step S8). In the present embodiment, estimation indexes are half-value widths W1, W2, etc. and areas A1, A2, etc. determined by the half-value widths of the peaks P1, P2, etc. As illustrated in FIG. 7, a half-value width is a width between two points serving as signal intensities that are 0.5 times of the signal intensity at the top of each peak. The area determined by a half-value width is represented by a value acquired by integrating signal intensities between two points defining the half-value width with each other. The estimation unit 10C associates the calculated half-value widths W1, W2, etc. and the areas A1, A2, etc. of the half-value widths with the peaks P1, P2, etc., and stores them in the peak list storage area generated at step S7. As a result, the peak list 132 in which the peaks determined to be derived from components that are identical to each other, the estimation indexes, and the values of m/z constituting the peaks are associated with each other is stored in the memory unit 13 or the RAM.

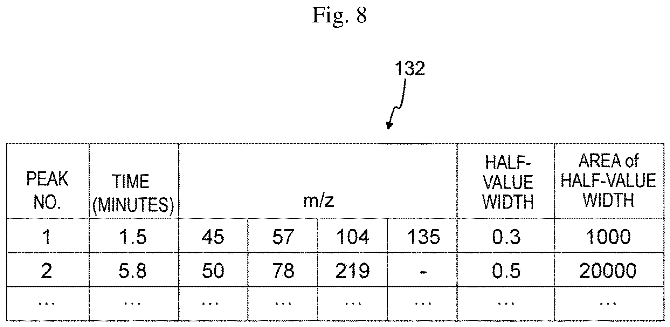

The peak list 132 is not limited to such a peak list described above. However, it is possible that the peak list is represented by data embodied in a table format as illustrated in FIG. 8. In the table, "Peak No." is a number identifying each of the peaks P1, P2, etc. sorted on the time axis, for example. A peak number corresponds to a thermal decomposition product that is a chemical compound having undergone component separation from a rubber composition or a volatile component having undergone component separation from a rubber composition. Furthermore, "Time (minutes)" is a retention time for each of the peaks P1, P2, etc. The value of "m/z" identifies a mass chromatogram constituting each of the peaks P1, P2, etc. The items of "Half-value width" and "Area of half-value width" are each of the half-value widths W1, W2, etc. and each of the areas A1, A2, etc. determined by half-value widths, respectively. That is, they represent data based on peaks identified for the mass chromatogram constituting the peaks P1, P2, etc. The item of "Area of half-value width" corresponds to a relative content of a thermal decomposition product or a volatile component in a rubber composition.

The estimation unit 10C then estimates at least either of a physical property and an odor degree of the rubber composition based on the data in the peak list 132 (step S9). There is no particular limitation in physical property to be estimated. Example physical properties include a glass transition temperature Tg, a viscosity coefficient, a storage elastic modulus, a loss elastic modulus, and a loss tangent (tan$\delta$). Furthermore, the odor degree is an index indicating the intensity of odor that a person feels from a rubber composition. The odor degree is a particularly important index when handling natural rubber containing a large amount of non-rubber component among rubber compositions. Note that at least some of the peaks P1, P2, etc. in the peak list 132 may be collated with peak data of a known TIC to identify a component that has derived the peak. When it has already been known a correlation between a content ratio of a component that has derived the peak in the rubber composition and a certain physical property of the rubber composition, the estimation unit 10C then extracts, from the peak list 132, data designated by the peak number corresponding to the component that has derived the peak, and estimates a certain physical property of the rubber composition based on the area determined by a half-value width. Alternatively, the estimation unit 10C extracts, from the peak list 132, data designated by the peak number corresponding to a certain odor component, and estimates an odor degree of the rubber composition based on the area determined by a half-value width. It is possible to perform the estimation using the method described in JP 6592985 B2, for example. However, steps S1 to S8 described above make it possible to separate peaks on a scale that is finer than conventional scales, achieving estimations at higher accuracy, compared with those performed conventionally. Note that the area determined by a half-value width is divided by the weight of a rubber composition to be subjected to the gas chromatograph-mass spectrometer, and thus normalized.

Furthermore, even when components that have derived the peaks P1, P2, etc. in the peak list 132 are not identified, collecting and studying the peak list 132 and pieces of data of physical properties for many rubber compositions make it possible to create a model representing a correlation between the areas determined by half-value widths of one or more certain peaks and certain physical properties. The model may be, for example, a model based on regression analysis or a machine learning model constructed through machine learning. It is possible that the estimation unit 10C applies the model, and estimates, based on the peak list 132 of an unknown rubber composition, a physical property of the rubber composition. There is no particular limitation in physical property to be estimated. Example physical properties include the glass transition temperature Tg, the viscosity coefficient, the storage elastic modulus, the loss elastic modulus, and the loss tangent (tan$\delta$), as described above.

Similarly, even when components that have derived the peaks P1, P2, etc. in the peak list 132 are not identified, collecting and studying the peak list 132 and pieces of data of odor degrees for many rubber compositions makes it possible to create a model representing a correlation between the areas determined by half-value widths of some or all of the peaks included in the peak list 132 and odor degrees. The model may be, for example, a model based on regression analysis or a machine learning model constructed through machine learning. It is possible to create data of odor degrees through sensory evaluation, for example. It is possible that the estimation unit 10C applies the model, and estimates, based on the peak list 132 of a rubber composition, an odor degree of the rubber composition.

The screen generation unit 10D generates a screen representing a result of the estimation at step S9, and causes the display unit 11 to display the generated screen (step S10). There is no particular limitation in the screen representing a result of estimation, as long as the screen displays at least one of a type and an estimation value of an estimated physical property and an odor degree. In addition, a screen may represent a result of estimation including the peak list 132 or graphics indicating data used for the estimation, among the peak list 132, for example.

<3. Features>

From the peak list 132 created in the embodiment described above, those minute peaks in mass chromatograms, which are normally ignored, are also extracted. Therefore, information of peaks derived from much more types of components in a rubber composition is acquired. As a result, it is possible to cover, in a wider range, many pieces of data of components derived from a rubber composition. Furthermore, in the peak list 132, peaks in mass chromatograms are separated at a scale finer than those in conventional TICs. Therefore, components derived from a rubber composition are more accurately reflected, making it possible to improve the accuracy in estimating a formulation, a physical property, and an odor degree of the rubber composition based on the peak list 132. Note that, if an operator carries out those tasks described above, much more efforts are required, while the number of pieces of data increases. In addition, it is extremely difficult to extract and separate minute peaks, making it impossible to create a peak list at accuracy allowing utilization for estimating a formulation, a physical property, and an odor degree of a rubber composition.

According to the embodiment described above, even when components that have derived the peaks P1, P2, etc. in the peak list 132 are not identified, it may be possible to estimate a physical property of a rubber composition based on the areas determined by half-value widths of peaks that appear at an identical point in time, as long as conditions for component separation and mass separation are unionized. Therefore, even when there are difficulties in acquiring data of peaks using a standard sample, it is possible to estimate a physical property of a rubber composition without identifying components that have derived the peaks P1, P2, etc.

According to the embodiment described above, it is possible to create a model representing a correlation between the areas determined by half-value widths and odor degrees of some or all of the peaks included in the peak list 132. That is, even when the correspondence between the peaks in the peak list 132 and odor components is not identified, it is possible to create such a model described above in which it is taken into account even a trace amount of an odor component in a rubber composition, and to estimate an odor degree in accordance with the model. Furthermore, even when estimating an odor degree of a rubber composition in accordance with a model representing a correlation between a content and an odor degree of an odor component that has been identified beforehand, similar to conventional cases, a content of an odor component is calculated at higher accuracy (as an area determined by a peak) in the embodiment described above. As a result, further improvement in estimation model is expected from those conventional estimation models.

<4. Modifications>

One embodiment of the present disclosure has been described above. However, the present disclosure is not limited to the embodiment described above. It is possible to modify the present disclosure in various ways without departing from the gist of the present disclosure. It is also possible to appropriately combine the main points of those modifications described below.

(1) In the embodiment described above, target data is outputted by a known gas chromatograph-mass spectrometer as data acquired by allowing a rubber composition to undergo component separation and mass separation. However, there is no particular limitation in such data, as long as the data is plotted in a space defined by a time axis, a signal intensity axis, and a mass component axis. For example, such data may be one outputted by a liquid chromatograph-mass spectrometer, instead of a gas chromatograph-mass spectrometer. Note that there is no particular limitation in detection method that a mass spectrometer applies. A desired detection method may be applied. Furthermore, a sample pretreatment method is not limited to the method exemplified in the embodiment described above. It is possible to appropriately change the sample pretreatment method.

(2) Step S2 may be performed after steps S3 and S4. That is, step S2 may be performed before step S5.

(3) In addition to information identifying mass components in chromatograms to which the peaks constituting the peaks P1, P2, etc. belong, the peak list 132 may include at least some of values of signal intensities at the tops, half-value widths, and the areas determined by the half-value widths of the peaks belonging to the chromatograms. That is, the peak identification unit 10B may calculate, for each peak identified at step S4, a signal intensity at the top, a half-value width, and the area determined by the half-value width, and may include them in the peak list 132. When estimating a physical property of a rubber composition, or when estimating an odor degree of the rubber composition, it is possible that the estimation unit 10C refers to the values as necessary. Note that information included in the peak list 132 is not limited to those introduced in the embodiment described above. It is possible to appropriately change such information. For example, information of the values of m/z, which identify mass chromatograms constituting the peaks P1, P2, etc., may be omitted from the peak list 132. One reason is that a combination of the values of m/z for peaks is useful for deriving more detailed information as a chemical compound, but is not essential for estimating a physical property and an odor degree of a rubber composition. Furthermore, as estimation indexes for estimating a physical property or an odor degree of a rubber composition, a width and an area determined by a peak to be calculated with another method may be used, in addition to a half-value width and the area determined by the half-value width.

(4) The methods for designating a grouping range at step S2, removing noise at step S3, and identifying a peak in signal intensity at step S4 are not limited to those methods introduced in the embodiment described above. It is possible adopt known methods.

(5) The indexes for determining whether peaks are derived from components that are identical to each other at step S6 are not limited to those introduced in the embodiment described above. Any indexes may be appropriately changed or added.

EXAMPLES

Examples of the present disclosure will now be described herein in detail. However, the present disclosure is not limited to the examples.

<Experiment 1>

A total of 100 types of rubber compositions that differ from each other in type and formulation of materials were produced. More specifically, after a polymer, a filler, a resin, and oil serving as raw materials of the rubber compositions were kneaded with a mixer, a vulcanization accelerator and sulfur were added in there. After the materials were further kneaded with a roll, the materials were vulcanized for 12 minutes at a temperature of 170°C. A sample at an amount of approximately 0.2 mg was collected from each of the rubber compositions. Each of the samples was subjected to a gas chromatograph-mass spectrometer. Three-dimensional data including a TIC and mass chromatograms was acquired. A range of mass components was set between 45 and 300. The mass chromatograms were acquired per one interval within the range. Based on the acquired data, prediction formulas for estimating the glass transition temperature Tg of a rubber composition from the area determined by a half-value width of a peak at a predetermined point in time S were created with two methods described below. Note that both the methods were used for two cases where the numbers of samples were 80 and 100.

[1] Based on the three-dimensional data including the TIC and the mass chromatograms, and by applying the method according to the embodiment described above, a peak list was created. In the peak list, a peak at the predetermined point in time S was identified. A data set of the area determined by a half-value width of the identified peak and the glass transition temperature Tg of the rubber composition was allowed to undergo regression analysis. A prediction formula for estimating the glass transition temperature Tg from the area determined by the half-value width of the peak was created. The area determined by the half-value width was normalized using the weight of the sample.

[2] Based on the TIC, an operator extracted peaks. A peak list was then created. In the peak list, a peak at the predetermined point in time S was identified. A data set of the area determined by a half-value width of the identified peak and the glass transition temperature Tg of the rubber composition was allowed to undergo regression analysis. A prediction formula for estimating the glass transition temperature Tg from the area determined by the half-value width of the peak was created. The area determined by the half-value width was normalized using the weight of the sample.

<Evaluation 1>

For the peak lists created with the methods [1] and [2] described above, the numbers of the extracted peaks were compared with each other. The numbers of the extracted peaks were obtained by averaging the numbers of the extracted peaks for all the samples. Furthermore, the prediction formulas for the glass transition temperature Tg, which were created with [1] and [2] described above, were evaluated. Specifically, data sets (predicted values and measured values) of Tg were each plotted on a plane defined by a horizontal axis indicating the glass transition temperature Tg that is predicted from the area determined by the half-value width of the peak at the predetermined point in time S and a vertical axis indicating the glass transition temperature Tg that was actually measured. The slopes of regression lines of the data sets were compared with each other. It is indicated that the closer the slope of the regression line to 1, the higher the accuracy of the prediction formula, and the farther the slope of the regression line from 1, the lower the accuracy of the prediction formula. The results are illustrated in Table 1.

[Table 1]

|  | [1] | | [2] | |
|---|---|---|---|---|
| Number of samples | 80 | 100 | 80 | 100 |
| Number of peaks | 448912 | 483102 | 35 | 48 |
| Slope | 0.845 | 0.897 | 0.241 | 0.311 |

As can be seen from the results, it is found that a much larger number of peaks were extracted when the method [1] was used, compared with the case when the method [2] was used. Furthermore, it is confirmed that it is possible to accurately predict the glass transition temperature Tg when the method [1] is used, compared with the case when the method [2] is used.

<Experiment 2>

A total of 30 types of natural rubber samples that differ from each other in combination of a production place and a manufacturing method were prepared. A sample at an amount of approximately 200 mg was collected from each of the samples. Each of the samples was subjected to a head space gas chromatograph-mass spectrometer. Three-dimensional data including a TIC and mass chromatograms was acquired, similar to Experiment 1. A range of mass components was set between 45 and 300. The mass chromatograms were acquired per one interval within the range. Based on the three-dimensional data including the TIC and the mass chromatograms, and by applying the method according to the embodiment described above, a peak list was created. For the 30 types of the samples, the average number of peaks acquired using the method was 448912.

For each of the samples, odor sensory evaluation was performed using the method disclosed in JP 6592985 B2. The odor degree was evaluated and rated to one point to six points. Regression analysis was performed based on the odor degrees and the areas determined by the half-value widths of all the peaks acquired for the samples. The odor degrees were used as objective variables, while the areas determined by the half-value widths of the peaks were used as explanatory variables. A prediction formula for estimating an odor degree was created. Note that the area determined by the half-value width was normalized using the weight of the sample.

<Evaluation 2>

A data set (a predicted value and a measured value) of the odor degree was plotted on a plane defined by a horizontal axis indicating an odor degree predicted from the area determined by the half-value width of the peak and a vertical axis indicating an odor degree acquired through odor sensory evaluation, in accordance with the prediction formula described above. The slope of the regression line of the data set was calculated. The calculated slope was 0.8 or higher. On the other hand, a prediction formula for estimating an odor degree was created using the method disclosed in JP 6592985 B2. The slope of the regression line of the data set (a predicted value and a measured value) was calculated in a similar manner. The calculated slope was 0.778. From this result, it has been confirmed that there is improvement in accuracy of estimating an odor degree when the method according to the embodiment described above is used.

Reference Signs List

| 1 | Analyzer |
| 10A | Acquisition unit |
| 10B | Peak identification unit |
| 10C | Estimation unit |
| 10D | Screen generation unit |
| 13 | Memory unit |

**Claims**

1.  A computer implemented
    analysis method for a rubber composition executed by one or more processors, the analysis method comprising:

    acquiring data acquired by allowing a rubber composition to undergo component separation and mass separation, the data being plotted in a space defined by a time axis, a signal intensity axis, and a mass component axis;
    identifying peaks in signal intensity for mass chromatograms corresponding to positions on the mass component axis;
    performing grouping within a range designated on the time axis for the peaks identified for the mass chromatograms,
    extracting a desired pair of the peaks having undergone the grouping, determining, when an index indicating a deviation between the peaks constituting the pair is equal to or lower than a predetermined threshold, the peaks constituting the pair as peaks derived from components that are identical to each other, and
    determining, when the index is above the threshold, the peaks constituting the pair as peaks derived from components that differ from each other; or
    extracting a desired pair of the peaks having undergone the grouping, determining, when an index indicating a deviation between the peaks constituting the pair is below the threshold, the peaks constituting the pair as peaks derived from components that are identical to each other, and
    determining, when the index is equal to or higher than the threshold, the peaks constituting the pair as peaks derived from components that differ from each other, and
    estimating at least either of a physical property and an odor degree of the rubber composition based on the peaks identified for the mass chromatograms.

2.  The analysis method according to claim 1, further comprising acquiring a chromatogram acquired by summing the signal intensities of the mass chromatograms that are present at an identical position on the time axis, and designating a range of the grouping to be performed based on the peaks identified in the chromatograms.

3.  The analysis method according to any one of claims 1 or 2, comprising estimating at least either of a certain physical property and an odor degree of the rubber composition based on areas determined by the peaks identified to be derived from the components that are identical to each other.

4. The analysis method according to any one of claims 1-3, comprising estimating at least either of a certain physical property and an odor degree of the rubber composition without identifying the components that have derived the peaks.

5. The analysis method according to any one of claims 1-4, wherein the physical property includes a glass transition temperature.

6. The analysis method according to any one of claims 1-5, wherein the data is acquired by a gas chromatograph-mass spectrometer.

7. The analysis method according to claim 6, wherein the data is acquired by a head space gas chromatograph-mass spectrometer.

8. An analyzer (1) for executing the analysis method for a rubber composition according to any one of claims 1 to 7, comprising:

   an acquisition unit (10A) that acquires data acquired by allowing a rubber composition to undergo component separation and mass separation, the data being plotted in a space defined by a time axis, a signal intensity axis, and a mass component axis;
   a peak identification unit (10B) that identifies peaks in signal intensity for mass chromatograms corresponding to positions on the mass component axis; and
   an estimation unit (10C) that estimates at least either of a physical property and an odor degree of the rubber composition based on the peaks identified for the mass chromatograms.

9. An analysis program for a rubber composition causing one or more processors to execute the analysis method of any one of claims 1 to 7.

10. Computer-readable medium, having stored thereon the analysis program of claim 9.

**Patentansprüche**

1. Computer-implementiertes Analyseverfahren für eine Kautschukzusammensetzung, ausgeführt durch einen oder mehrere Prozessoren, wobei das Analyseverfahren umfasst:

   Erfassen von Daten, die erfasst werden, indem eine Kautschukzusammensetzung einer Komponententrennung und Massentrennung unterzogen wird, wobei die Daten in einem durch eine Zeitachse, eine Signalintensitätsachse und eine Massenkomponentenachse definierten Raum aufgetragen werden;
   Identifizieren von Peaks in der Signalintensität für Massen-Chromatogramme, welche Positionen auf der Massenkomponentenachse entsprechen;
   Durchführen einer Gruppierung innerhalb eines auf der Zeitachse festgelegten Bereichs für die Peaks, die für die Massen-Chromatogramme identifiziert wurden,
   Extrahieren eines gewünschten Paars der Peaks, die der Gruppierung unterzogen worden sind, Bestimmen, wenn ein Index, der eine Abweichung zwischen den Peaks, die das Paar bilden, anzeigt, gleich oder unter einem vorgegebenen Schwellenwert ist, dass die Peaks, die das Paar bilden, Peaks sind, welche von Komponenten stammen, die miteinander identisch sind, und
   Bestimmen, wenn der Index oberhalb des Schwellenwertes ist, dass die Peaks, die das Paar bilden, Peaks sind, welche von Komponenten stammen, die sich voneinander unterscheiden; oder
   Extrahieren eines gewünschten Paars der Peaks, die der Gruppierung unterzogen worden sind, Bestimmen, wenn ein Index, der eine Abweichung zwischen den Peaks, die das Paar bilden, anzeigt, unterhalb des Schwellenwerts ist, dass die Peaks, die das Paar bilden, Peaks sind, welche von Komponenten stammen, die miteinander identisch sind, und
   Bestimmen, wenn der Index gleich oder über dem Schwellenwert ist, dass die Peaks, die das Paar bilden, Peaks sind, welche von Komponenten stammen, die sich voneinander unterscheiden, und
   Schätzen von mindestens einem von einer physikalischen Eigenschaft und einem Grad an Geruch der Kautschukzusammensetzung auf Grundlage der für die Massen-Chromatogramme identifizierten Peaks.

2. Analyseverfahren nach Anspruch 1, zudem umfassend das Erfassen eines Chromatogramms, das erfasst wird, indem die Signalintensitäten der Massen-Chromatogramme, die an einer identischen Position auf der Zeitachse vorliegen, summiert werden, und
Festlegen eines Bereichs der durchzuführenden Gruppierung auf Grundlage der in den Chromatogrammen identifizierten Peaks.

3. Analyseverfahren nach einem der Ansprüche 1 oder 2, umfassend das Schätzen von mindestens einem von einer bestimmten physikalischen Eigenschaft und einem Grad an Geruch der Kautschukzusammensetzung auf Grundlage von Flächen, die durch die Peaks bestimmt sind, welche identifiziert sind als von den Komponenten stammend, die miteinander identisch sind.

4. Analyseverfahren nach einem der Ansprüche 1 bis 3, umfassend das Schätzen von mindestens einem von einer bestimmten physikalischen Eigenschaft und einem Grad an Geruch der Kautschukzusammensetzung, ohne die Komponenten zu identifizieren, von denen die Peaks stammen.

5. Analyseverfahren nach einem der Ansprüche 1 bis 4, wobei die physikalische Eigenschaft eine Glasübergangstemperatur umfasst.

6. Analyseverfahren nach einem der Ansprüche 1 bis 5, wobei die Daten durch ein Gaschromatograph-Massenspektrometer erfasst werden.

7. Analyseverfahren nach Anspruch 6, wobei die Daten durch ein Dampfraum-Gaschromatograph-Massenspektrometer erfasst werden.

8. Analysegerät (1) zum Ausführen des Analyseverfahrens für eine Kautschukzusammensetzung nach einem der Ansprüche 1 bis 7, umfassend:

eine Erfassungseinheit (10A), die Daten erfasst, indem eine Kautschukzusammensetzung einer Komponententrennung und Massentrennung unterzogen wird, wobei die Daten in einem durch eine Zeitachse, eine Signalintensitätsachse und eine Massenkomponentenachse definierten Raum aufgetragen werden;
eine Peak-Identifizierungseinheit (10B), die Peaks in der Signalintensität für Massen-Chromatogramme identifiziert, welche Positionen auf der Massenkomponentenachse entsprechen; und
eine Schätzungseinheit (10C), die mindestens eines von einer physikalischen Eigenschaft und einem Grad an Geruch der Kautschukzusammensetzung auf Grundlage der für die Massen-Chromatogramme identifizierten Peaks schätzt.

9. Analyseprogramm für eine Kautschukzusammensetzung, welches einen oder mehrere Prozessoren dazu veranlasst, das Analyseverfahren nach einem der Ansprüche 1 bis 7 auszuführen.

10. Computer-lesbares Medium, auf dem das Analyseprogramm nach Anspruch 9 gespeichert ist.

**Revendications**

1. Procédé d'analyse mis en œuvre par ordinateur pour une composition de caoutchouc exécuté par un ou plusieurs processeurs, le procédé d'analyse comprenant :

l'acquisition de données acquises en permettant à une composition de caoutchouc de subir une séparation de composants et une séparation de masse, les données étant portées sur un graphe dans un espace défini par un axe de temps, un axe d'intensité de signal, et un axe de composants de masse,
l'identification de pics d'intensité de signal pour des chromatogrammes de masse correspondant aux positions sur l'axe de composants de masse, la réalisation d'un groupement dans une plage désignée sur l'axe de temps pour les pics identifiés pour les chromatogrammes de masse,
l'extraction d'une paire souhaitée des pics ayant subi le groupement, la détermination, lorsqu'un indice indiquant une déviation entre les pics constituant la paire est inférieur ou égal à un seuil prédéterminé, les pics constituant la paire comme pics dérivés des composants qui sont identiques les uns aux autres, et

la détermination, lorsque l'indice est au-dessus du seuil, des pics constituant la paire comme pics dérivés de composants qui diffèrent les uns des autres ; ou

l'extraction d'une paire souhaitée des pics ayant subi le groupement, la détermination, lorsqu'un indice indice indiquant une déviation entre les pics constituant la paire est au-dessous du seuil, les pics constituant la paire comme pics dérivés de composants qui sont identiques les uns aux autres, et

la détermination, lorsque l'indice est supérieur ou égal au seuil, des pics constituant la paire comme pics dérivés de composants qui diffèrent les uns des autres, et

l'estimation d'au moins un d'une propriété physique et d'un degré d'odeur de la composition de caoutchouc sur la base des pics identifiés pour les chromatogrammes de masse.

2. Procédé d'analyse selon la revendication 1, comprenant en outre l'acquisition d'un chromatogramme acquis en sommant les intensités de signal des chromatogrammes de masse qui sont présents à une position identique sur l'axe de temps, et

la désignation d'une plage du groupement à effectuer sur la base des pics identifiés dans les chromatogrammes.

3. Procédé d'analyse selon l'une quelconque des revendications 1 ou 2, comprenant l'estimation d'au moins un d'une certaine propriété physique et d'un degré d'odeur de la composition de caoutchouc sur la base des zones déterminées par les pics identifiés pour être dérivés des composants qui sont identiques les uns aux autres.

4. Procédé d'analyse selon l'une quelconque des revendications 1 à 3, comprenant l'estimation d'au moins un d'une certaine propriété physique et d'un degré d'odeur de la composition de caoutchouc sans identifier les composant qui ont fait dériver les pics.

5. Procédé d'analyse selon l'une quelconque des revendications 1 à 4, dans lequel la propriété physique comprend une température de transition vitreuse.

6. Procédé d'analyse selon l'une quelconque des revendications 1 à 5, dans lequel les données sont acquises par un spectromètre de masse-chromatographe de gaz.

7. Procédé d'analyse selon la revendication 6, dans lequel les données sont acquises par un spectromètre de masse-chromatographe de gaz à espace de tête.

8. Analyseur (1) pour exécuter le procédé d'analyse pour une composition de caoutchouc selon l'une quelconque des revendications 1 à 7, comprenant une unité d'acquisition (10A) qui acquiert les données acquises en permettant à une composition de caoutchouc de subir une séparation de composants et une séparation de masse, les données étant portées sur un graphe dans un espace défini par un axe de temps, un axe d'intensité de signal, et un axe de composants de masse,

une unité d'identification de pic (10B) qui identifie les pics de l'intensité de signal pour les chromatogrammes de masse correspondant aux positions sur l'axe de composants de masse ; et

une unité d'estimation (10C) qui estime au moins l'un d'une propriété physique et d'un degré d'odeur de la composition de caoutchouc sur la base des pics identifiés pour les chromatogrammes de masse.

9. Programme d'analyse pour une composition de caoutchouc entraînant un ou plusieurs processeurs à exécuter le programme d'analyse selon l'une quelconque des revendications 1 à 7.

10. Support lisible sur ordinateur, ayant stocké dessus le programme d'analyse selon la revendication 9.

Fig. 1

Fig. 2

Fig. 3

```
          ┌───────────────┐
          │     START     │
          └───────┬───────┘
                  │
                  ▼
       ┌─────────────────────┐
       │    ACQUIRE DATA     │  ⌇ S1
       └──────────┬──────────┘
                  │
                  ▼
       ┌─────────────────────┐
       │ DESIGNATE GROUPING  │  ⌇ S2
       │        RANGE        │
       └──────────┬──────────┘
                  │
                  ▼
       ┌─────────────────────┐
       │  REMOVE NOISE FROM  │  ⌇ S3
       │    CHROMATOGRAM     │
       └──────────┬──────────┘
                  │
                  ▼
       ┌─────────────────────┐
       │ IDENTIFY PEAK IN    │  ⌇ S4
       │  SIGNAL INTENSITY   │
       └──────────┬──────────┘
                  │
                  ▼
       ┌─────────────────────┐
       │      GROUPING       │  ⌇ S5
       └──────────┬──────────┘
                  │
                  ▼
    ┌──────────────────────────┐
    │ DETERMINE WHETHER THEY   │  ⌇ S6
    │ ARE DERIVED FROM         │
    │ COMPONENTS THAT ARE      │
    │ IDENTICAL TO EACH OTHER  │
    └──────────────┬───────────┘
                   │
                   ▼
       ┌─────────────────────┐
       │ SUM PEAKS DETERMINED│  ⌇ S7
       │ TO BE IDENTICAL TO  │
       │     EACH OTHER      │
       └──────────┬──────────┘
                  │
                  ▼
       ┌─────────────────────┐
       │  CREATE PEAK LIST   │  ⌇ S8
       └──────────┬──────────┘
                  │
                  ▼
       ┌─────────────────────┐
       │  ESTIMATE PHYSICAL  │  ⌇ S9
       │ PROPERTY/ODOR DEGREE│
       └──────────┬──────────┘
                  │
                  ▼
       ┌─────────────────────┐
       │ GENERATE AND DISPLAY│  ⌇ S10
       │       SCREEN        │
       └──────────┬──────────┘
                  │
                  ▼
          ┌───────────────┐
          │      END      │
          └───────────────┘
```

Fig. 4

Fig. 5

Fig. 6

Fig. 7

Fig. 8

132

| PEAK NO. | TIME (MINUTES) | m/z | | | | HALF-VALUE WIDTH | AREA of HALF-VALUE WIDTH |
|---|---|---|---|---|---|---|---|
| 1 | 1.5 | 45 | 57 | 104 | 135 | 0.3 | 1000 |
| 2 | 5.8 | 50 | 78 | 219 | - | 0.5 | 20000 |
| ... | ... | ... | ... | ... | ... | ... | ... |

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2013160599 A
- JP 6592985 B
- CN 114043651 A
- CN 113176354 A

**Non-patent literature cited in the description**

- **X. CHEN et al.** *Environ. Sci. Pollut. Res.*, 2022, vol. 29, 9685-9692